# EUROPEAN PATENT APPLICATION

(11) **EP 4 230 239 A1**
(43) Date of publication of application: **23.08.2023**
(21) Application number: 23155593.9
(22) Date of filing: 08.02.2023
(51) Int. Cl.: A61M 5/142, A61M 5/168, A61M 39/02

(54) **IMPLANTABLE PUMP WITH ACCUMULATOR FOR BOLUS DELIVERY**

(30) Priority: 16.02.2022 US 202217673620
(71) Applicant: Medtronic, Inc., Minneapolis, MN 55432-5604 (US)
(72) Inventor: HAASE, James M., Maplewood (US); DREW, Touby A., Golden Valley (US)
(74) Representative: Zimmermann & Partner Patentanwälte mbB

(57) **Abstract**

An implantable pump configured to enable tuning of a delivery velocity of a fixed quantity of medicament. The implantable pump including a pump, an accumulator and a valve configured to enable tuning of a delivery velocity of a fixed quantity of medicament, wherein operating the pump with the valve continuously in the open state enables a steady-state delivery of medicament at a first velocity, and wherein closing of the valve enables the pump to at least partially fill the accumulator and subsequent opening of the valve enables the at least partially filled accumulator to dispense medicament, thereby delivering a bolus of medicament at a second velocity, wherein the second velocity is greater than the first velocity.

## Description

### TECHNICAL FIELD

The present technology is generally related to implantable medical devices, and more particularly to implantable medical pumps configured to enable low-volume steady-state medicament delivery as well as larger volume boluses of medicament, thereby enabling a single device to deliver medicament over a wider range of velocities and volumes.

### BACKGROUND

Implantable medical devices, such as implantable medical pumps, are useful in managing the delivery and dispensation of prescribed therapeutic agents, nutrients, drugs, infusates such as antibiotics, blood clotting agents, analgesics and other fluid or fluid like substances (collectively "infusate" or "infusates") to patients in volume- and time-controlled doses as well as through boluses. Such implantable pumps are particularly useful for treating diseases and disorders that require regular or chronic (i.e., long-term) pharmacological intervention, including tremor, spasticity, multiple sclerosis, Alzheimer's disease, Parkinson's disease, amyotrophic lateral sclerosis (ALS), Huntington's disease, cancer, epilepsy, chronic pain, urinary or fecal incontinence, sexual dysfunction, obesity, and gastroparesis, to name just a few. Depending upon their specific designs and intended uses, implantable pumps are well adapted to administer infusates to specific areas within the vasculatures and central nervous system, including the subarachnoid, epidural, intrathecal, and intracranial spaces or provide access to those spaces for aspiration.

Providing access to the cerebrospinal fluid for the administration of infusates or aspiration of fluid has a number of important advantages over other forms of infusate administration. For example, oral administration is often not workable because the systematic dose of the substance needed to achieve the therapeutic dose at the target site may be too large for the patient to tolerate without adverse side effects. Also, some substances simply cannot be absorbed in the gut adequately for a therapeutic dose to reach the target site. Moreover, substances that are not lipid soluble may not cross the blood-brain barrier adequately if needed in the brain. In addition, infusion of substances from outside the body requires a transcutaneous catheter or access with a hypodermic needle, which results in other risks such as infection or catheter dislodgment. Further, implantable pumps avoid the problem of patient noncompliance, namely the patient failing to take the prescribed drug or therapy as instructed.

Such implantable pumps are typically implanted at a location within the body of a patient (typically a subcutaneous region in the lower abdomen) and are connected to a catheter configured to deliver infusate to a selected delivery site in the patient. The catheter is generally configured as a flexible tube with a lumen running the length of the catheter to a selected delivery site in the body, such as the intracranial or subarachnoid space.

Some implantable pumps administer medicament through operation of a peristaltic pumping mechanism, which pumps fluid from an expandable fluid reservoir to a selected delivery site in the body in an extremely precise, relatively low-volume steady-state. For example, the SynchroMed^{™} line of implantable pumps (manufactured and sold by Medtronic PLC) employs such a peristaltic pumping mechanism. Other implantable pumps employ a much less precise, valve mechanism configured to selectively release medicament from a pressurized reservoir into a patient in a series of larger volume boluses. For example, the Prometra^{™} line of implantable pumps (manufactured and sold by Flowonix Medical, Inc.) employs such a valve mechanism. Although efforts have been made to increase customization of pump parameters to enable medicament delivery over a wider range of volumes while still maintaining a high degree of precision, further improvements in medicament delivery customization are always desirable. The present disclosure addresses this concern.

### SUMMARY OF THE DISCLOSURE

The techniques of this disclosure generally relate to optionally pulsatile flow generating implantable systems and pumps including a peristaltic pump configured to precisely deliver steady state flow of medicament and a combination accumulator and valve configured to selectively deliver larger boluses, thereby enabling a single device to deliver medicament over a wider range of velocities and volumes. As an additional benefit, implantable pumps of the present disclosure may use the accumulator and valve to study the effects of pressure decay after medicament release relevant to calibration and measurement system pressures, to assist in the detection and diagnosis of catheter occlusion, dislodgment, kink in other system issues.

One embodiment of the present disclosure provides an implantable pump configured to enable tuning of a delivery velocity of a fixed quantity of medicament, including a pump, an accumulator fluidly coupled to the pump, and a valve fluidly coupled to the accumulator, the valve configured to transition between an open state in a closed state, wherein operating the pump with the valve continuously in the open state enables a steady-state delivery of medicament at a first velocity, and wherein closing of the valve enables the pump to at least partially fill the accumulator, and subsequent opening of the valve enables the at least partially filled accumulator to dispense medicament, thereby delivering a bolus of medicament at a second velocity, wherein the second velocity is greater than the first velocity.

In one embodiment, the pump is a peristaltic pump. In one embodiment, the pump is configured to pump medicament at a rate of between about 0 µL/hr and about 1 µL/hr. In one embodiment, the accumulator defines a vessel separated into a first portion and a second portion by a bladder. In one embodiment, the accumulator is configured to temporally store at least about 0.25 µL of medicament. In one embodiment, the implantable pump further includes a computing device configured to sense an electromotive force voltage of the pump. In one embodiment, the implantable pump further includes a pressure sensor configured to sense a pressure of medicament downstream of the pump.

Another embodiment of the present disclosure provides an implantable pump including a pump, an accumulator and a valve configured to enable tuning of a delivery velocity of a fixed quantity of medicament, wherein operating the pump with the valve continuously in the open state enables a steady-state delivery of medicament at a first velocity, and wherein closing of the valve enables the pump to at least partially fill the accumulator and subsequent opening of the valve enables the at least partially filled accumulator to dispense medicament, thereby delivering a bolus of medicament at a second velocity, wherein the second velocity is greater than the first velocity.

In one embodiment, the pump is a peristaltic pump. In one embodiment, the pump is configured to pump medicament at a steady-state rate of between about 0 µL/hr and about 1 µL/hr. In one embodiment, the accumulator is configured to be charged with a bolus of at least about 0.25 µL of medicament. In one embodiment, the implantable pump further includes a computing device configured to sense oscillations in an electromotive force voltage of the pump. In one embodiment, the implantable pump further includes a pressure sensor configured to sense a pressure of medicament downstream of the pump, wherein the pressure sensor activates upon the detection of damped oscillations via the sensed electromotive force voltage.

Yet another embodiment of the present disclosure provides an implantable pump with occlusion detection including a peristaltic pump, an accumulator fluidly coupled to the peristaltic pump, a valve fluidly coupled to the accumulator, the valve configured to transition between an open state and a closed state, and a computing device configured to sense an electromotive force voltage from the peristaltic pump, wherein closing of the valve enables the pump to at least partially charge the accumulator, and subsequent opening of the valve enables the at least partially charged accumulator to discharge, wherein the computing device senses the electromotive force voltage during discharge of the accumulator to infer a medicament pressure decay.

In one embodiment, the pump is configured to pump medicament at a steady-state rate of between about 0 µL/hr and about 1 µL/hr. In one embodiment, the accumulator is configured to be charged with a bolus of at least about 0.25 µL of medicament. In one embodiment, the computing device is configured to sense oscillations in the electromotive force voltage. In one embodiment, the computing device is configured to determine if the oscillations are damped in reference to a model electromotive force voltage curve. In one embodiment, the implantable pump further includes a pressure sensor configured to sense a pressure of medicament downstream of the pump. In one embodiment, the pressure sensor activates upon the detection of damped oscillations in the sensed electromotive force voltage.

The details of one or more aspects of the disclosure are set forth in the accompanying drawings and the description below. Other features, objects, and advantages of the techniques described in this disclosure will be apparent from the description in the drawings, and from the claims.

Further disclosed herein is an implantable pump configured to enable tuning of a delivery velocity of a fixed quantity of medicament. The implantable pump including a pump, an accumulator and a valve configured to enable tuning of a delivery velocity of a fixed quantity of medicament, wherein operating the pump with the valve continuously in the open state enables a steady-state delivery of medicament at a first velocity, and wherein closing of the valve enables the pump to at least partially fill the accumulator and subsequent opening of the valve enables the at least partially filled accumulator to dispense medicament, thereby delivering a bolus of medicament at a second velocity, wherein the second velocity is greater than the first velocity.

### BRIEF DESCRIPTION OF THE DRAWINGS

The disclosure can be more completely understood in consideration of the following detailed description of various embodiments of the disclosure, in connection with the accompanying drawings, in which:
FIG. 1 is a schematic view depicting a medical system configured to enable targeted drug delivery over a wide range of velocities and volumes, including steady-state delivery of medicament, periodic or pulsed boluses of medicament, or combinations thereof, in accordance with an embodiment of the disclosure.
FIGS. 2A-B are cross sectional views depicting an implantable device configured to enable targeted drug delivery over a wide range of velocities and volumes, in accordance with an embodiment of the disclosure.
FIG. 3 is a block diagram of an implantable device and programmer configured to enable tuning of a delivery velocity of a fixed quantity of medicament, in accordance with an embodiment of the disclosure.
FIG. 4A is a schematic view depicting a peristaltic pump, accumulator and valve configured to enable steady-state delivery of medicament, periodic or pulsed boluses of medicament, or combinations thereof over a wide range of velocities and volumes, wherein the valve is in a closed position to enable charging of the accumulator with the pump, in accordance with an embodiment of the disclosure.
FIG. 4B is the peristaltic pump, accumulator and valve of FIG. 4A, wherein the valve is in the opened position to enable bolus discharge drug delivery of the accumulator or steady-state drug delivery via the pump, in accordance with an embodiment of the disclosure.
FIG. 5 is a graphical representation depicting a medicament delivery profile representing a volume of medicament delivery under a steady-state delivery profile and a bolus delivery profile over a period of time, in accordance with an embodiment of the disclosure.
FIG. 6A depicts dispersion of medicament within cerebrospinal fluid of a patient at a relatively low velocity, in accordance with an embodiment of the disclosure.
FIG. 6B depicts dispersion of medicament within cerebrospinal fluid of a patient at a higher velocity than that depicted in FIG. 6A.
FIG. 7 is a graphical representation depicting a pump EMF voltage over a series of samples following a bolus delivery, in accordance with an embodiment of the disclosure.

While embodiments of the disclosure are amenable to various modifications and alternative forms, specifics thereof shown by way of example in the drawings will be described in detail. It should be understood, however, that the intention is not to limit the disclosure to the particular embodiments described. On the contrary, the intention is to cover all modifications, equivalents, and alternatives falling within the spirit and scope of the subject matter as defined by the claims.

### DETAILED DESCRIPTION

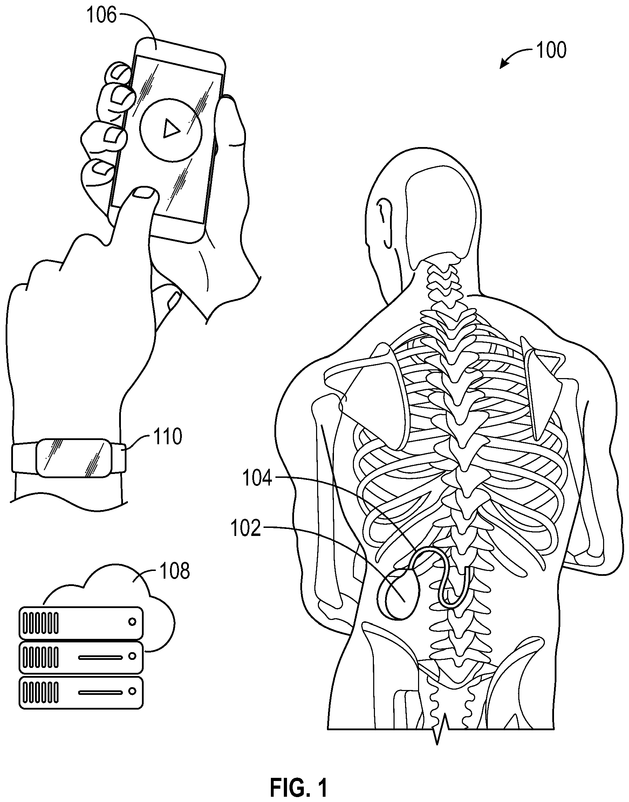

Referring to FIG. 1, a medical system 100 comprising an implantable medical device 102 configured to enable targeted drug delivery over a wide range of velocities and volumes, including steady-state delivery of medicament, periodic, pulsed boluses of medicament, or combinations thereof, in accordance with an embodiment of the disclosure. In embodiments, the medical system 100 can include an implantable catheter 104, which can be in fluid communication with the implantable medical device 102, which can be an implantable pump or smart port, configured to dispense infusate over an extended period of time. As depicted, the implantable device 102 can be implanted within the body of a patient, for example, in an interior torso cavity or in proximity to the patient's ribs or cranially for the introduction of infusate into the patient (e.g., within an intrathecal space, intracranial space, pulmonary artery, etc.) for targeted delivery of infusate. In some embodiments, the implantable device 102 can be placed subcutaneously, and can be held in position by sutures or other retaining features.

Various example embodiments of implantable medical devices, systems and methods are described herein providing a wider range of velocities and volumes of infusate delivery. Although specific examples of implantable medical pumps are provided, it is to be appreciated that the concepts disclosed herein are extendable to other types of implantable devices. It is also to be appreciated that the term "clinician" refers to any individual that can prescribe and/or program a therapeutic regimen with any of the example embodiments described herein or alternative combinations thereof. Similarly, the term "patient" or "subject," as used herein, is to be understood to refer to an individual or object in which the infusate delivery is to occur, whether human, animal, or inanimate. Various descriptions are made herein, for the sake of convenience, with respect to the procedures being performed by a clinician on a patient or subject (the involved parties collectively referred to as a "user" or "users") while the disclosure is not limited in this respect.

In some embodiments, the medical system 100 can further include an optional external programmer 106 and optional server 108 configured to communicate with the implantable device 102. In some embodiments, the programmer 106 can be a handheld, wireless portable computing device, such as a cellular telephone, tablet, dedicated implantable device programmer, or the like. Further, in some embodiments, the medical system 100 can include one or more external physiological sensors 110, which can be in communication with the implantable device 102, optional external programmer 106, and optional server 108. In one embodiment, one or more physiological sensors 110 can be incorporated into the implantable device 102 or the external programmer 106. In one embodiment, a physiological sensor 110 can be worn by the patient (e.g., a smart watch, wristband tracker, sensors embedded in clothing, etc.), carried by the patient (e.g., a smart phone, mobile computing device, etc.), or positioned in proximity to the patient (e.g., a stationary monitor, etc.). Examples of physiological sensors 110 include a heart rate monitor, pulse oximeter, respiratory sensor, perspiration sensor, posture orientation sensor, motion sensor, accelerometer, or the like.

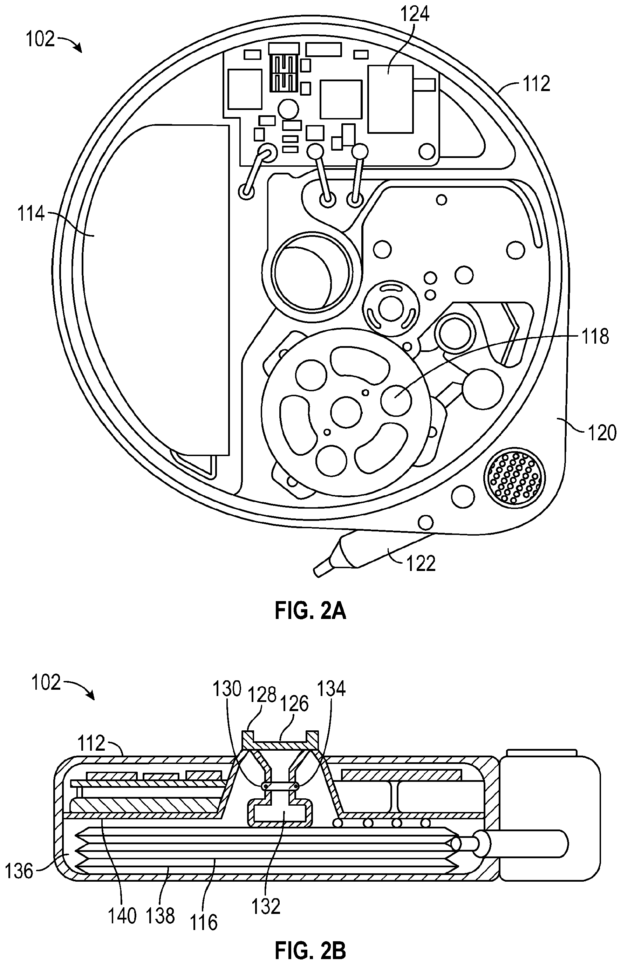

Referring to FIGS. 2A-B, cross sectional views of an implantable device 102 configured to enable targeted drug delivery over a wide range of velocities and volumes are depicted in accordance with an embodiment of the disclosure. The implantable device 102 can generally include a housing 112, power source 114, fluid reservoir 116, pump 118, accumulator 120, valve 122, and computing device 124. The housing 112 can be constructed of a material that is biocompatible and hermetically sealed, such as titanium, tantalum, stainless steel, plastic, ceramic, or the like.

The fluid reservoir 116 can be carried by the housing 112 and can be configured to contain infusate. In one embodiment, infusate within the reservoir 116 can be accessed via an access port 126. Accordingly, the access port 126 can be utilized to refill, aspirate, or exchange fluid within the reservoir 116. In some embodiments, the access port 126 can include one or more positional markers 128, for example in the form of a tactile protrusion, one or more lights or LEDs to illuminate through tissue of the patient, an acoustic device to confirm location of the access port 126, and/or one or more wireless location/orientation sensors to aid in positioning of a refilling device relative to the implantable device 102.

In some embodiments, the access port 126 can include a septum 130 configured to seal a port chamber 132 relative to an exterior of the housing 112. The septum 130 can be constructed of a silicone rubber or other material having desirable self-sealing and longevity characteristics. The port chamber 132 can be in fluid communication with the reservoir 116. In one embodiment, the access port 126 can further include an optional needle detection sensor 134, for example in the form of a mechanical switch, resonant circuit, ultrasonic transducer, voltmeter, ammeter, ohmmeter, pressure sensor, flow sensor, capacitive probe, acoustic sensor, and/or optical sensor configured to detect and confirm the presence of an injection needle of a refilling apparatus.

The reservoir 116 can include a flexible diaphragm 136. The flexible diaphragm 136, alternatively referred to as a bellows, which can be substantially cylindrical in shape and can include one or more convolutions configured to enable the flexible diaphragm 138 to expand and contract between an extended or full position and an empty position. In one embodiment, the flexible diaphragm 138 can divide the reservoir 116 into an infusate chamber containing liquid infusate (within the flexible diaphragm 138), and a vapor chamber 140 (surrounding the flexible diaphragm 138). As the infusate chamber is filled with infusate, the flexible diaphragm 138 extends downwardly (with reference to FIG. 2B) toward a bottom portion of the housing 112 until it has reached a maximum volume or some other desired degree of fullness. Alternatively, as the infusate chamber is aspirated, the flexible diaphragm 138 contracts upwardly toward a top portion of the housing 112 until the infusate chamber reaches a minimum volume. In one embodiment, the flexible diaphragm 138 can have a compression spring rate which tends to naturally bias the flexible diaphragm 138 towards an expanded position.

The pump 118, accumulator 120 and valve 122 can be carried by the housing 112. The pump 118 can be in fluid communication with the reservoir 116 and can be in electrical communication with the computing device 120. The pump 118 can be any pump sufficient for pulling infusate from the reservoir 116 four downstream delivery, such as a peristaltic pump, piston pump, a pump powered by a stepper motor or rotary motor, a pump powered by an AC motor, a pump powered by a DC motor, electrostatic diaphragm, piezioelectric motor, solenoid, shape memory alloy, or the like. Infusate from the pump 118 can flow through the accumulator 120, which can be configured to retain a quantity of infusate when the valve 122 is in the closed position, and subsequently release the infusate when the valve 122 is opened.

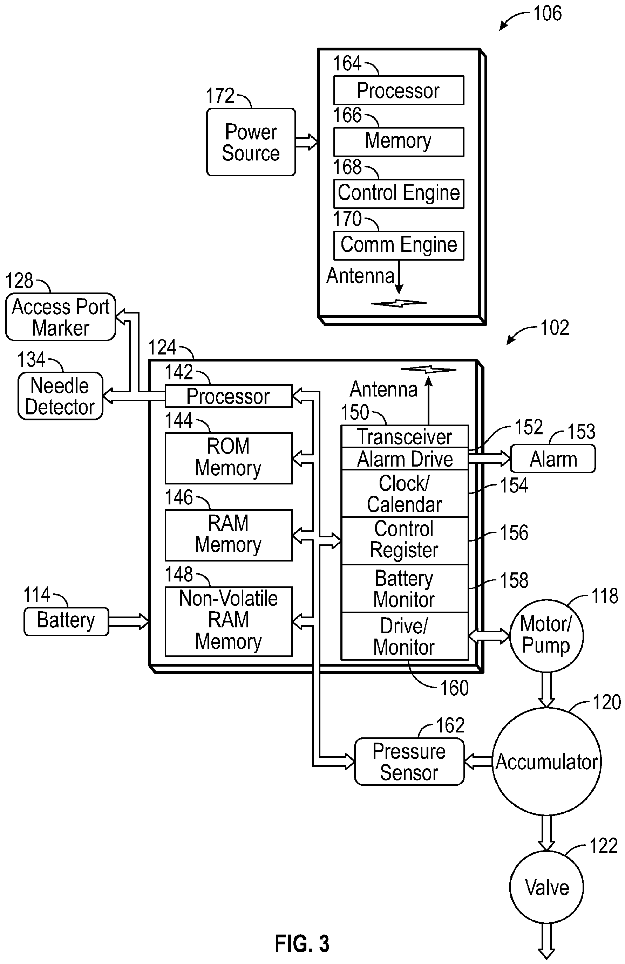

Referring to FIG. 3, a block diagram of an implantable device 102 and programmer 106 configured to enable steady-state delivery of medicament, periodic, pulse boluses of medicament, or combinations thereof over a wider range of velocities and volumes, is depicted in accordance with an embodiment of the disclosure. The implantable device 102 can include a computing device 124, which can be carried in the housing 112 (as depicted in FIG. 2A) and can be in electrical communication with the pump 118, valve 122 and power source 114. The power source 114 can be a battery, such as a rechargeable lithium-ion battery, nickel cadmium battery, or the like. The power source 114, which can be monitored via the battery monitor 158, can be carried in the housing 112 to power the pump 118, valve 122 and computing device 124. Control of the pump 118 and valve 122 can be directed by a drive/monitor element 160.

The computing device 124 can include a processor 142, memory 144, 146 and 148, and transceiver circuitry 150. In one embodiment, the processor 142 can be a microprocessor, logic circuit, Application-Specific Integrated Circuit (ASIC) state machine, gate array, controller, or the like. The computing device 124 can generally be configured to control delivery of infusate according to programmed parameters or a specified treatment protocol. The programmed parameters or specified treatment protocol can be stored in the memory 144, 146 and 148 for specific implementation by a control register 156. A clock/calendar element 154 can maintain system timing for the computing device 124. In one embodiment, an alarm drive 152 can be configured to activate one or more notification, alert or alarm features, such as an illuminated, auditory or vibratory alarm 153. In some embodiments, the processor 142 can be configured to selectively activate the needle detection sensor 134 and access port marker 128, prior to a physical attempt to insert a needle of the refill device into the access port 126 of the implantable device 102. Further, in some embodiments, the processor 142 can be configured to receive input from the drive/monitor element 160 and optional pressure sensor 162, which can serve as an aid in detecting occlusions and generally monitoring a pressure decay of infusate within the accumulator 120 (or tubing generally associated with the catheter 104) following an opening of valve 122.

The transceiver circuitry 150 can be configured to receive information from and transmit information to the one or more physiological sensors 110, external programmer 106, and server 108. The implantable device 102 can be configured to receive programmed parameters and other updates from the external programmer 106, which can communicate with the implantable device 102 through well-known techniques such as wireless telemetry, Bluetooth, or one or more proprietary communication schemes (e.g., Tel-M, Tel-C, etc.). In some embodiments, the external programmer 106 can be configured for exclusive communication with one or more implantable devices 102. In other embodiments, the external programmer 106 can be any computing platform, such as a mobile phone, tablet or personal computer. In some embodiments, the implantable device 102 and external programmer 106 can further be in communication with a cloud-based server 108. The server 108 can be configured to receive, store and transmit information, such as program parameters, treatment protocols, drug libraries, and patient information, as well as to receive and store data recorded by the implantable device 102.

In embodiments, estimation of a volume of remaining medicament within a fluid reservoir of an implantable medical device 102 can be performed, at least partially by the programmer 106. In one embodiment, the programmer 106 or components thereof can comprise or include various modules or engines, each of which is constructed, programmed, configured, or otherwise adapted to autonomously carry out a function or set of functions. The term "engine" as used herein is defined as a real-world device, component, or arrangement of components implemented using hardware, such as by an application specific integrated circuit (ASIC) or field programmable gate array (FPGA), for example, or as a combination of hardware and software, such as by a microprocessor system and a set of program instructions that adapt the engine to implement the particular functionality, which (while being executed) transform the microprocessor system into a special-purpose device. An engine can also be implemented as a combination of the two, with certain functions facilitated by hardware alone, and other functions facilitated by a combination of hardware and software. In certain implementations, at least a portion, and in some cases, all, of an engine can be executed on the processor(s) of one or more computing platforms that are made up of hardware (e.g., one or more processors, data storage devices such as memory or drive storage, input/output facilities such as network interface devices, video devices, keyboard, mouse or touchscreen devices, etc.) that execute an operating system, system programs, and application programs, while also implementing the engine using multitasking, multithreading, distributed (e.g., cluster, peer-peer, cloud, etc.) processing where appropriate, or other such techniques. Accordingly, each engine can be realized in a variety of physically realizable configurations, and should generally not be limited to any particular implementation exemplified herein, unless such limitations are expressly called out. In addition, an engine can itself be composed of more than one sub-engines, each of which can be regarded as an engine in its own right. Moreover, in the embodiments described herein, each of the various engines corresponds to a defined autonomous functionality; however, it should be understood that in other contemplated embodiments, each functionality can be distributed to more than one engine. Likewise, in other contemplated embodiments, multiple defined functionalities may be implemented by a single engine that performs those multiple functions, possibly alongside other functions, or distributed differently among a set of engines than specifically illustrated in the examples herein.

In some embodiments, the programmer 106 can include a processor 164, memory 166, a control engine 168, a communications engine 170, and a power source 172. Processor 164 can include fixed function circuitry and/or programmable processing circuitry. Processor 164 can include any one or more of a microprocessor, a controller, a DSP, an ASIC, an FPGA, or equivalent discrete or analog logic circuitry. In some examples, the processor 162 can include multiple components, such as any combination of one or more microprocessors, one or more controllers, one or more DSPs, one or more ASICs, or one or more FPGAs, as well as other discrete or integrated logic circuitry. The functions attributed to processor 164 herein may be embodied as software, firmware, hardware or any combination thereof.

The memory 166 can include computer-readable instructions that, when executed by processor 164 cause ECU 120 to perform various functions. Memory 166 can include volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media. Control engine 168 can include instructions to control the components of the programmer 106 and instructions to selectively control the implantable medical device 102.

The communications engine 170 can include any suitable hardware, firmware, software, or any combination thereof for communicating with other components of the medical device 102 and/or external devices. Under the control of processor 164, the communication engine 170 can receive downlink telemetry from, as well as send uplink telemetry to one or more external devices (e.g., the implantable medical device 102, etc.) using an internal or external antenna. In addition, communication engine 170 can facilitate communication with a networked computing device and/or a computer network 108. For example, communications engine 170 can receive updates to instructions for control engine 168 from one or more external devices. In another example, communications engine 170 can transmit data regarding the state of system 100 to one or more one or more external devices.

Power source 172 is configured to deliver operating power to the components of the programmer 106. Power source 172 can include a battery and a power generation circuit to produce the operating power. In some examples, the battery is rechargeable to allow extended operation. Power source 172 can include any one or more of a plurality of different battery types. In some embodiments, the programmer 106 can further include an external power supply port.

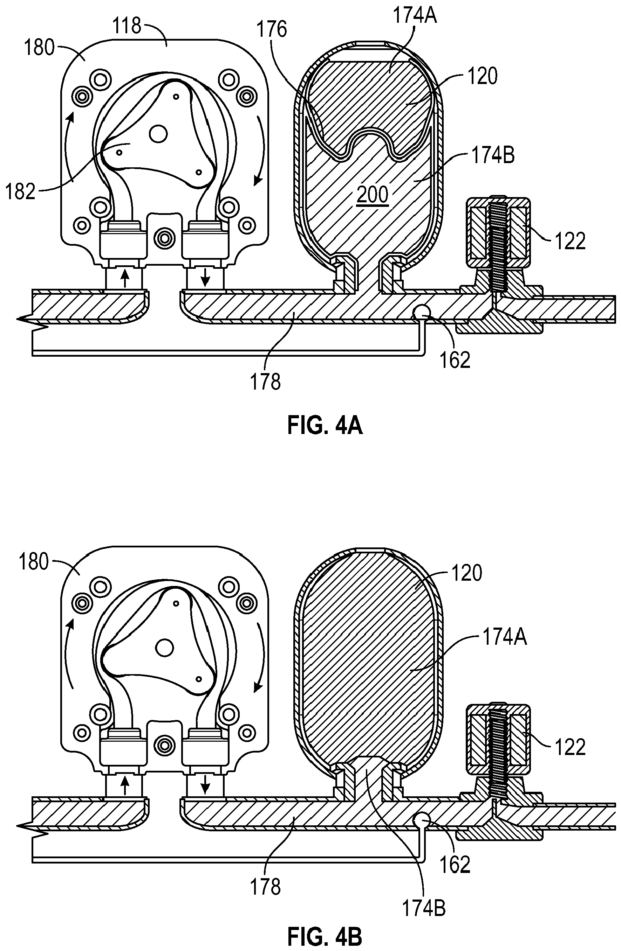

With additional reference to FIGS. 4A-B, a peristaltic pump 118, accumulator 120 and valve 122 configured to enable steady-state delivery of medicament, periodic, pulse boluses of medicament, or combinations thereof over a wider range of velocities and volumes, are depicted in accordance with an embodiment of the disclosure. As depicted in FIG. 4A, when the valve 122 is in the closed position, continued pumping of the peristaltic pump 118 causes medicament to collect within the accumulator 120.

For example, in some embodiments, the accumulator 120 can define a vessel 174A/B partitioned by a bladder 176, thereby separating the vessel into a first portion 174A and a second portion 174B, with the first portion 174A filled with a compressible fluid (e.g., nitrogen or the like); although other types of accumulators are also contemplated. Accordingly, as the pump 118 continues to operate, pressurized medicament 200 flows through conduit 178 and into the second portion 174B, thereby displacing the bladder 176 and compressing the compressible fluid within first portion 174A of the accumulator 120. Thereafter, the pressurized medicament 200 can be retained within the accumulator 120 for subsequent delivery as desired. In some embodiments, a pressure of the medicament 200 can be measured by the pressure sensor 162.

As further depicted in FIG. 4B, upon transitioning the valve 122 to the open position, medicament 200 retained within the accumulator 120 can be evacuated, for example by the expansion of first portion 174A under a pressure of the compressible fluid, thereby causing the medicament 200 to flow outwardly through the conduit 178 and into the catheter 104 for targeted delivery within the patient. Moreover, the bolus can be delivered without sacrificing precision, as the accumulator 120 can be filled with the same volumetric precision delivered by the pump 118 during steady-state conditions. Alternatively, operating the pump 118 with the valve 122 opened enables a steady-state delivery of medicament.

Accordingly, selective activation of the pump 118 and valve 122 can enable a wider range of medicament delivery velocities and volumes, without sacrificing precision, particularly in comparison to pumps of the prior art. Specifically, embodiments of the present disclosure are configured to enable a desired quantity of medicament to be delivered through a relatively low velocity steady-state condition, or through higher velocity boluses, thereby affecting a different spread pattern of the medicament at the target site. In some embodiments, steady-state medicament can be delivered in a range of between about 0 µL/hr to about 1 µL/hr (e.g., 1/100 µL/hr, 1/50 µL/hr, 1/20 µL/hr, 1/10 µL/hr, etc.), and bolus medicament delivery can be delivered in quantities of 0.25 µL or more (e.g., 0.5 µL, 1 µL, 1.5 µL, 2.0 µL, 2.5 µL, etc.). Other steady-state and bolus delivery rates and amounts are also contemplated.

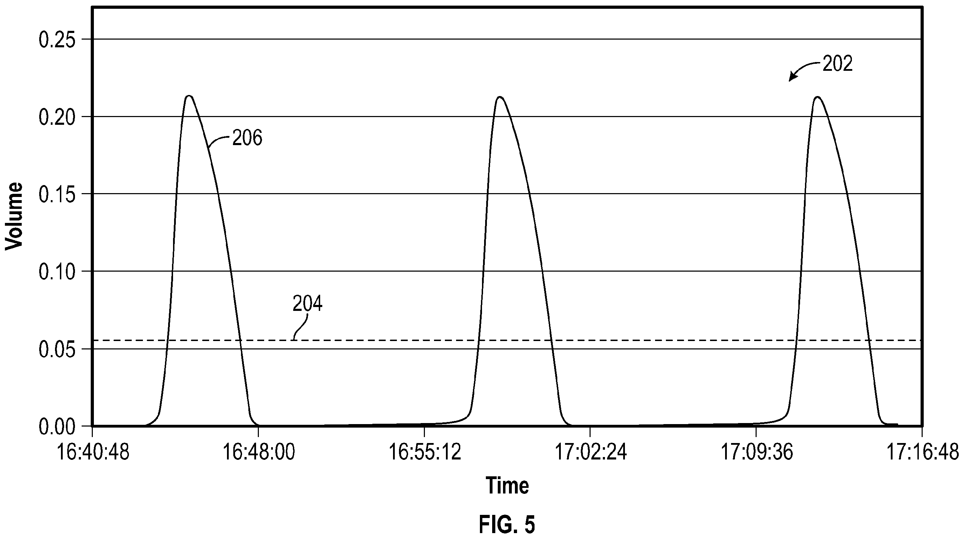

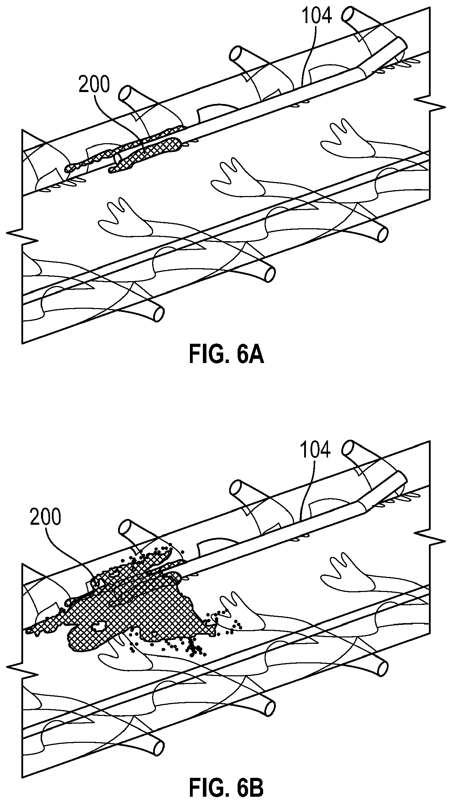

With reference to FIG. 5, a medicament delivery profile 202 graphically representing a volume of medicament delivery under a steady-state delivery profile 204 and a bolus delivery profile 206 over a period of time. As depicted, the y-axis represents the volume of medicament delivery in µL, while the x-axis represents the time of day. With additional reference to FIGS. 6A-B, the effects of the medicament delivery under the different profiles 204, 206 can be observed. For example, with specific reference to FIG. 6A, under the steady-state delivery profile 204, the medicament 200 is ejected from the catheter 104 at a relatively low velocity, such that the medicament is dispersed into bodily fluid in relative close proximity to the end of the catheter 104. By contrast, with specific reference to FIG. 6B, under a bolus delivery profile 206, the medicament 200 is ejected from the catheter 104 at a relatively higher velocity, such that the medicament is dispersed into bodily fluid a further distance from the end of the catheter 104.

Thus, even though the different profiles 204, 206 ultimately deliver the same quantity of medicament, tailoring the profile, which in turn affects fluid velocity, can have a significant impact on delivery and dispersion of the medicament. For example, it may be found that a patient responds better to lower velocity infusions during certain conditions (e.g., times of day, levels of physical activity, etc.), whereas during other conditions the patient responds better to higher velocity infusions. Accordingly, embodiments of the present disclosure enable a clinician to tailor medicament delivery profiles and infusion velocities to improve the therapeutic effects of the treatment regimen. Moreover, should a catheter 104 unintentionally shift or move from its original position, rather than undergoing a surgical procedure to replace the catheter 104, a clinician may alter the delivery profile (e.g., increase or decrease infusion velocity) to affect delivery of the medicament to the original targeted site.

As an additional benefit, embodiments of the present disclosure can use the pump 118, accumulator 120 and valve 122 to study the effects of pressure decay after medicament release to assist in the detection and diagnosis of catheter occlusion, dislodgment, kink in other 100 system issues. For example, in some embodiments, the pressure can be directly sensed via a pressure sensor 162 in communication with the computing device 124. Alternatively, or in addition to the pressure sensor 162, medicament pressure and pressure decay can be inferred from a sensed electromotive force (EMF) voltage of the pump 116.

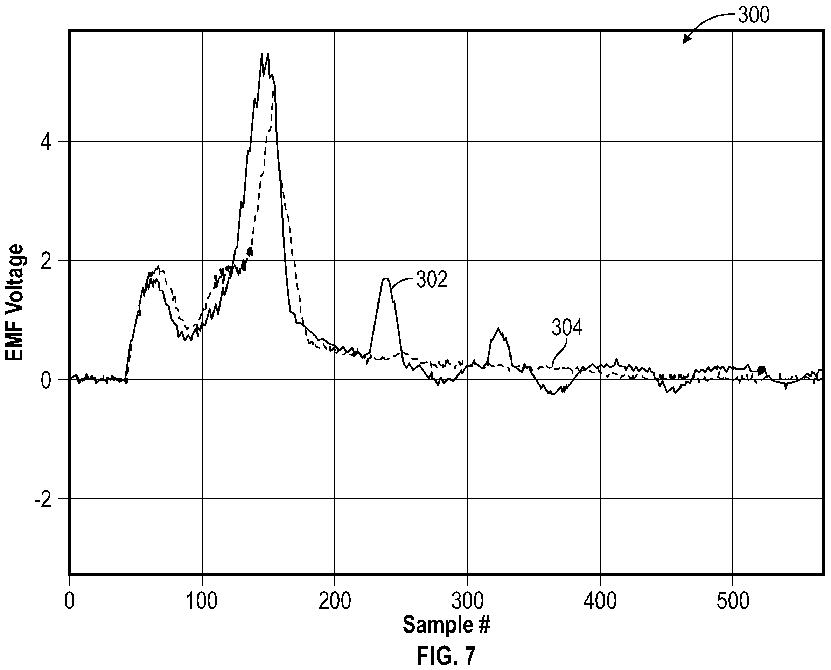

FIG. 7 graphically represents a pump EMF voltage over a series of samples 300 following a bolus delivery. As depicted, the y-axis represents the EMF voltage (e.g., mV), while the x-axis represents a series of samples (e.g., on a scale of several thousand samples per second). Pump EMF voltage can be used to indirectly determine pressure by noting movement in the rotor of the pump after power has been removed. For example, with additional reference to FIGS. 3 & 4A-B, drive currents to the stator 180 are selectively applied and removed by the drive/monitor element 160. Through computing device 124, a resulting EMF voltage can be sensed in each of the stator coils 180 after the drive currents are removed. From these EMF voltages, a position of the valve rotor 182 can be determined.

The rotor 182 will naturally come to rest at an equilibrium position determined by the magnetic forces between the stator 180 and rotor 182, as well as external forces (e.g., pressurized medicament within conduit 178. As depicted in FIG. 7, a first plot 302 depicts oscillations in the EMF voltage as the peristaltic pump 118 rocks back and forth slightly to settle in an equilibrium position. By contrast, the second plot 304 depicts less pronounced oscillations in the EMF voltage, as movement of the peristaltic pump 118 is significantly dampened by pressurized medicament trapped within the downstream conduit 178. Accordingly, dampened oscillations in sensed EMF voltage can be indicative of slow pressure decay (e.g., occlusion, kinked tubing, system issues, etc.).

In some embodiments, pressure sensing can be primarily or exclusively performed through monitoring of the pump 118, thereby significantly reducing power usage, as activation of the pressure sensor 162 naturally consumes battery life. Accordingly, in some embodiments, the pump 118 can operate in a series of pulses (e.g., rotations or partial rotations) until any medicament stored within the accumulator 120 has been completely expelled, such that movement of the pump rotor can be sensed between pulses, thereby inferring downstream medicament pressure decay during medicament administration. In some embodiments, abnormal damping in sensed the EMF voltages can trigger activation of the pressure sensor 162 for potential verification of impeded or partially impeded pressure decay.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Further disclosed herein is the subject-matter of the following clauses:
1. An implantable pump configured to enable tuning of a delivery velocity of a fixed quantity of medicament, the implantable pump comprising:
   a pump;
   an accumulator fluidly coupled to the pump; and
   a valve fluidly coupled to the accumulator, the valve configured to transition between an open state and a closed state,
   wherein operating the pump with the valve continuously in the open state enables a steady-state delivery of medicament at a first velocity, and
   wherein closing of the valve enables the pump to at least partially fill the accumulator, and subsequent opening of the valve enables the at least partially filled accumulator to dispense medicament, thereby delivering a bolus of medicament at a second velocity, wherein the second velocity is greater than the first velocity.
2. The implantable pump of clause 1, wherein the pump is a peristaltic pump.
3. The implantable pump of clause 1 or 2, wherein the pump configured to pump medicament at a rate of between about 0 µL/hr and about 1 µL/hr.
4. The implantable pump of clause 1 or of any of the preceding clauses, wherein the accumulator defines a vessel separated into a first portion and a second portion by a bladder.
5. The implantable pump of clause 1 or of any of the preceding clauses, wherein the accumulator is configured to temporally store at least about 0.25 µL of medicament.
6. The implantable pump of clause 1 or of any of the preceding clauses, further comprising a computing device configured to sense an electromotive force voltage of the pump.
7. The implantable pump of clause 1 or of any of the preceding clauses, further comprising a pressure sensor configured to sense a pressure of medicament downstream of the pump.
8. An implantable pump comprising:
   a pump, an accumulator and a valve configured to enable tuning of a delivery velocity of a fixed quantity of medicament, wherein operating the pump with the valve continuously in the open state enables a steady-state delivery of medicament at a first velocity, and wherein closing of the valve enables the pump to at least partially fill the accumulator and subsequent opening of the valve enables the at least partially filled accumulator to dispense medicament, thereby delivering a bolus of medicament at a second velocity, wherein the second velocity is greater than the first velocity.
9. The implantable pump of clause 8, wherein the pump is a peristaltic pump.
10. The implantable pump of clause 8 or 9, wherein the pump configured to pump medicament at a steady-state rate of between about 0 µL/hr and about 1 µL/hr.
11. The implantable pump of clause 8 or of any of clauses 8 to 10, wherein the accumulator is configured to be charged with a bolus of at least about 0.25 µL of medicament.
12. The implantable pump of clause 8 or of any of clauses 8 to 11, further comprising a computing device configured to sense oscillations in an electromotive force voltage of the pump.
13. The implantable pump of clause 8 or of any of clauses 8 to 12, further comprising a pressure sensor configured to sense a pressure of medicament downstream of the pump, wherein the pressure sensor activates upon the detection of damped oscillations the sensed electromotive force voltage.
14. An implantable pump with occlusion detection, the implantable pump comprising:
   a peristaltic pump;
   an accumulator fluidly coupled to the peristaltic pump; and
   a valve fluidly coupled to the accumulator, the valve configured to transition between an open state and a closed state; and
   a computing device configured sense an electromotive force voltage from the peristaltic pump,
   wherein closing of the valve enables the pump to at least partially charge the accumulator, and subsequent opening of the valve enables the at least partially charged accumulator to discharge, wherein the computing device senses the electromotive force voltage during discharge of the accumulator to infer a medicament pressure decay.
15. The implantable pump of clause 14, wherein the pump configured to pump medicament at a steady-state rate of between about 0 µL/hr and about 1 µL/hr.
16. The implantable pump of clause 14 or 15, wherein the accumulator is configured to be charged with a bolus of at least about 0.25 µL of medicament.
17. The implantable pump of clause 14 or of any of clauses 14 to 16, wherein the computing device is configured to sense oscillations in the electromotive force voltage.
18. The implantable pump of clause 14 or of any of clauses 14 to 17, wherein the computing device is configured to determine if the oscillations are damped in in reference to a model electromotive force voltage curve.
19. The implantable pump of clause 14 or of any of clauses 14 to 18, further comprising a pressure sensor configured to sense a pressure of medicament downstream of the pump.
20. The implantable pump of clause 14 or of any of clauses 14 to 19, wherein the pressure sensor activates upon the detection of damped oscillations the sensed electromotive force voltage.

## Claims

1. An implantable pump configured to enable tuning of a delivery velocity of a fixed quantity of medicament, the implantable pump comprising:
a pump;
an accumulator fluidly coupled to the pump; and
a valve fluidly coupled to the accumulator, the valve configured to transition between an open state and a closed state,
wherein operating the pump with the valve continuously in the open state enables a steady-state delivery of medicament at a first velocity, and
wherein closing of the valve enables the pump to at least partially fill the accumulator, and subsequent opening of the valve enables the at least partially filled accumulator to dispense medicament, thereby delivering a bolus of medicament at a second velocity, wherein the second velocity is greater than the first velocity.

2. The implantable pump of claim 1, wherein the pump is a peristaltic pump, and/or
wherein the pump configured to pump medicament at a rate of between about 0 µL/hr and about 1 µL/hr.

3. The implantable pump of one of the claims 1 to 2, wherein the accumulator defines a vessel separated into a first portion and a second portion by a bladder, and/or
wherein the accumulator is configured to temporally store at least about 0.25 µL of medicament.

4. The implantable pump of one of the claims 1 to 3, further comprising a computing device configured to sense an electromotive force voltage of the pump.

5. The implantable pump of one of the claims 1 to 4, further comprising a pressure sensor configured to sense a pressure of medicament downstream of the pump.

6. An implantable pump comprising:
a pump, an accumulator and a valve configured to enable tuning of a delivery velocity of a fixed quantity of medicament, wherein operating the pump with the valve continuously in the open state enables a steady-state delivery of medicament at a first velocity, and wherein closing of the valve enables the pump to at least partially fill the accumulator and subsequent opening of the valve enables the at least partially filled accumulator to dispense medicament, thereby delivering a bolus of medicament at a second velocity, wherein the second velocity is greater than the first velocity.

7. The implantable pump of claim 6, wherein the pump is a peristaltic pump, and/or wherein the pump configured to pump medicament at a steady-state rate of between about 0 µL/hr and about 1 µL/hr.

8. The implantable pump of one of the claims 6 to 7, wherein the accumulator is configured to be charged with a bolus of at least about 0.25 µL of medicament.

9. The implantable pump of one of the claims 6 to 8, further comprising a computing device configured to sense oscillations in an electromotive force voltage of the pump.

10. The implantable pump of one of the claims 6 to 9, further comprising a pressure sensor configured to sense a pressure of medicament downstream of the pump, wherein the pressure sensor activates upon the detection of damped oscillations the sensed electromotive force voltage.

11. An implantable pump with occlusion detection, the implantable pump comprising:
a peristaltic pump;
an accumulator fluidly coupled to the peristaltic pump; and
a valve fluidly coupled to the accumulator, the valve configured to transition between an open state and a closed state; and
a computing device configured sense an electromotive force voltage from the peristaltic pump,
wherein closing of the valve enables the pump to at least partially charge the accumulator, and subsequent opening of the valve enables the at least partially charged accumulator to discharge, wherein the computing device senses the electromotive force voltage during discharge of the accumulator to infer a medicament pressure decay.

12. The implantable pump of claim 11, wherein the pump configured to pump medicament at a steady-state rate of between about 0 µL/hr and about 1 µL/hr, and/or
wherein the accumulator is configured to be charged with a bolus of at least about 0.25 µL of medicament.

13. The implantable pump of one of the claims 11 to 12, wherein the computing device is configured to sense oscillations in the electromotive force voltage, and/or
wherein the computing device is configured to determine if the oscillations are damped in in reference to a model electromotive force voltage curve.

14. The implantable pump of one of the claims 11 to 13, further comprising a pressure sensor configured to sense a pressure of medicament downstream of the pump.

15. The implantable pump of one of the claims 11 to 14, wherein the pressure sensor activates upon the detection of damped oscillations the sensed electromotive force voltage.
